# EUROPÄISCHE PATENTSCHRIFT

(11) **EP 2 957 309 B1**
(45) Veröffentlichungstag und Bekanntmachung des Hinweises auf die Patenterteilung: **13.03.2019**
(21) Anmeldenummer: 14172547.3
(22) Anmeldetag: 16.06.2014
(51) Int. Cl.: A61M 5/30

(54) **Zylinder-Kolben-Einheit mit mindestens einer an den Zylinderboden angrenzenden Düse**
Cylinder-piston unit with at least one nozzle adjacent to the cylinder base
Unité cylindre-piston ayant au moins une buse installée au fond du cylindre

(43) Veröffentlichungstag der Anmeldung: 23.12.2015
(73) Patentinhaber: LTS LOHMANN Therapie-Systeme AG, 56626 Andernach (DE)
(72) Erfinder: Hoffmann, Hans-Rainer, 56566 Neuwied (DE)
(74) Vertreter: Thämer, Wolfgang

(56) Entgegenhaltungen:
- EP-B1- 1 848 480
- DE-A1-102007 055 405
- DE-A1-102011 007 314
- US-A- 3 788 315
- US-A1- 2008 269 687

## Beschreibung

Die Erfindung betrifft eine Zylinder-Kolben-Einheit eines Einweginjektors mit einem Zylinder und mit einem in diesem abgedichtet geführten Kolben, wobei der Zylinder und der Kolben einen Verdrängungsraum begrenzen und wobei mindestens eine Düse mit einer dem Verdrängungsraum zugewandten Eintrittsfläche und mit einer der Umgebung zugewandten Austrittsfläche den Verdrängungsraum mit der Umgebung verbindet, wobei die Düse mindestens eine Gerade umgreift, die die Eintrittsfläche und die Austrittsfläche schneidet und wobei die Kolbenhubrichtung des Kolbens in Richtung des Verdrängungsraums und eines Zylinderbodens des Zylinders orientiert ist.

Aus der EP 1 848 480 B1 ist eine Zylinder-Kolben-Einheit mit einem Düsenblock bekannt. Im Düsenblock zweigen einzelne Düsen von einem zentralen Zufuhrkanal ab.

Aus der US 3,788,315 A ist eine Zylinder-Kolben-Einheit bekannt, bei der Düsen den Zylinderboden durchdringen. Die Düsenlängsrichtungen liegen hierbei innerhalb eines Kegels mit einem Öffnungswinkel von 10 Grad zur Kolbenhubrichtung. Die Eintrittsfläche und die Austrittsfläche der einzelnen Düsen liegen im Zylinderboden.

Die in der DE 10 2007 055 405 A1 offenbarte Ampulleneinheit hat einen Düsenblock aus Stahl, der mittels eines Dichtungselements angedichtet mechanisch an einem Grundkörper fixiert ist. In der kugelförmig ausgebildeten Spitze des Düsenblocks sind Düsen angeordnet.

Der vorliegenden Erfindung liegt die Problemstellung zugrunde, eine Zylinder-Kolben-Einheit mit einer verbesserten Ausbringung zu entwickeln.

Diese Problemstellung wird mit den Merkmalen des Hauptanspruchs gelöst. Dazu hat der Zylinder eine Mantelfläche mit einem an den Zylinderboden angrenzenden Düsenbereich, in dem der Verdrängungsraum mittels der Düse mit der Umgebung verbunden ist. An den Zylinderboden grenzt die Eintrittsfläche an. Außerdem schließt die parallel zu der genannten Gerade orientierte, von der Eintrittsfläche zur Austrittsfläche gerichtete Düsenlängsrichtung mit der Kolbenhubrichtung in einer gemeinsamen Ebene einen Winkel zwischen 45 Grad und 105 Grad ein.

Weitere Einzelheiten der Erfindung ergeben sich aus den Unteransprüchen und den nachfolgenden Beschreibungen schematisch dargestellter Ausführungsbeispiele.
- Figur 1:: Teillängsschnitt einer Zylinder-Kolben-Einheit;
- Figur 2:: Querschnitt A-A der Zylinder-Kolben-Einheit aus Figur 1;
- Figur 3:: Variante einer Zylinder-Kolben-Einheit;
- Figur 4:: Detail aus Figur 3.

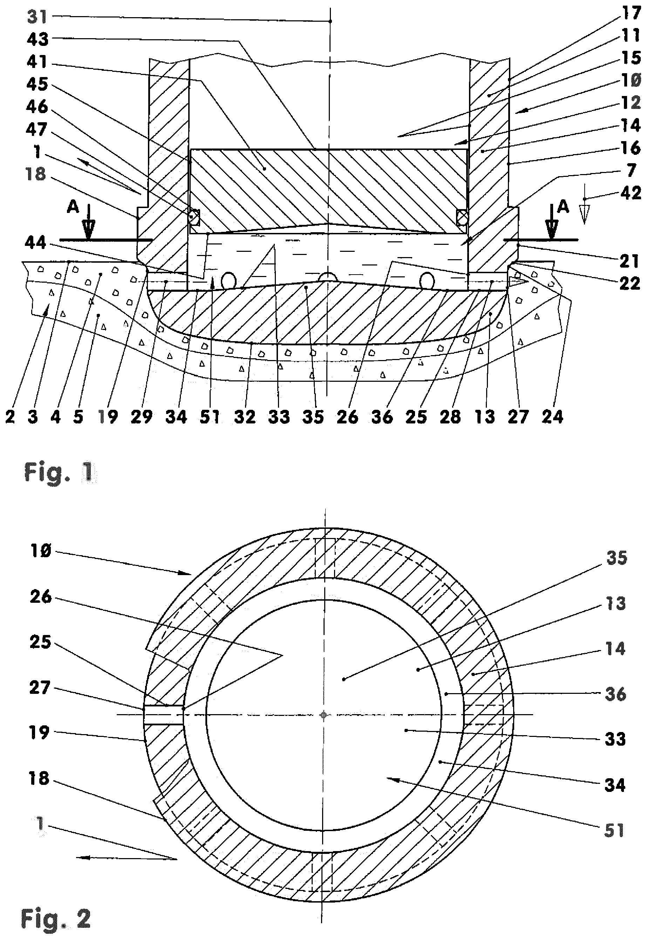

Die Figuren 1 und 2 zeigen einen Teillängsschnitt und einen Querschnitt einer Zylinder-Kolben-Einheit (10). In den Darstellungen sind unsichtbare Linien gestrichelt dargestellt. Die dargestellte Zylinder-Kolben-Einheit (10) wird beispielsweise in einem Einweginjektor eingesetzt, mit dem z.B. wirkstoffhaltige Lösungen (7) in die Haut eines Patienten eingebracht werden. Die Zylinder-Kolben-Einheit (10) ist hierfür beispielsweise mit einem eine Antriebsvorrichtung umfassenden Gehäuse des Einweginjektors verrastet.

Die Zylinder-Kolben-Einheit (10) umfasst einen Zylinder (11) und einen im Zylinder (11) geführten Kolben (41). Der Zylinder (11) und der Kolben (41) begrenzen im Zylinderinnenraum (12) einen an einen Zylinderboden (13) angrenzenden Verdrängungsraum (51). Hierbei ist die Kolbenhubrichtung (42) des Kolbens (41) in Richtung des Verdrängungsraums (51) und des Zylinderbodens (13) gerichtet.

Der Zylinder (11) hat einen Zylindermantel (14), dessen Innenwandung (15) dem Zylinderinnenraum (12) zugewandt ist und dessen hierzu z.B. koaxiale, abschnittsweise definierte Mantelfläche (16) der Umgebung (1) zugewandt ist. Die Innenwandung (15) ist beispielsweise zylindrisch oder kegelstumpfförmig ausgebildet.

Die Mantelfläche (16) weist in diesem Ausführungsbeispiel drei Abschnitte (17 - 19) auf. Ein erster Abschnitt ist ein z.B. zylindrisch ausgebildeter Mantelabschnitt (17). Er grenzt beispielsweise an einen hier nicht dargestellten Befestigungsbereich an, mit dem die Zylinder-Kolben-Einheit (10) im Einweginjektor befestigt ist.

Der zweite Abschnitt (18) ist ein Auflagering (18). In den Darstellungen der Figuren 1 und 2 ist er an den Zylinder (11) angeformt. Er kann aber auch auf diesen aufgesetzt sein. Im Ausführungsbeispiel hat der Auflagering (18) einen kreisförmigen Außendurchmesser und steht in radialer Richtung über den dritten Abschnitt (19) über. Dieser Überstand beträgt beispielsweise zwischen einem Millimeter und zehn Millimetern. Die Umfangsfläche (21) des Auflagerings (18) kann glatt oder strukturiert ausgebildet sein. Der Auflagering (18) kann auch einzelne Segmente aufweisen. Die in Richtung des Zylinderbodens (13) orientierte Unterseite (22) des Auflagerings (18) ist im Ausführungsbeispiel als Kegelstumpf ausgebildet. Die Unterseite (22) kann aber auch eine Planfläche umfassen. Der Übergang der Unterseite (22) zum dritten Abschnitt (19) ist im Ausführungsbeispiel als umlaufende Hohlkehle (24) ausgebildet.

Der dritte Abschnitt (19), der im Ausführungsbeispiel an den Zylinderboden (13) angrenzt, ist ein Düsenbereich (19). Er ist beispielsweise zylindrisch ausgebildet. Es ist aber auch denkbar, den Düsenbereich (19) kegelstumpfförmig auszubilden, wobei die gedachte Kegelspitze z.B. in Richtung des Zylinderbodens (13) versetzt zum Düsenbereich (19) liegt.

Im Düsenbereich (19) ist der Verdrängungsraum (51) mittels mindestens einer den Zylinder (11) durchdringenden Düse (25) mit der Umgebung (1) verbunden. Im Ausführungsbeispiel weist die Zylinder-Kolben-Einheit (10) acht radial angeordnete Düsen (25) auf, die jeweils eine dem Verdrängungsraum (51) zugewandte Eintrittsfläche (26) und eine der Umgebung (1) zugewandte Austrittsfläche (27) haben. Die Querschnittsfläche der einzelnen Düse (25) kann in der von der Eintrittsfläche (26) zur Austrittsfläche (27) gerichteten Düsenlängsrichtung (28) konstant sein oder zunehmen. Beispielsweise kann die Düse (25) trichterförmig ausgebildet sein. Die einzelnen Düsen (25) können auch unterschiedlich ausgebildet und/oder ausgerichtet sein. Der Durchmesser der einzelnen Düse (25) an der Austrittsfläche (27) beträgt beispielsweise zwischen 0,15 Millimeter und 0,3 Millimeter. Der Durchmesser der einzelnen Düse (25) an der Eintrittsfläche (26) beträgt z.B. 0,15 Millimeter. Die Länge der einzelnen Düse (25) entspricht im Ausführungsbeispiel der Dicke des Zylindermantels (14) im Düsenbereich (19). Sie beträgt z.B. einen Millimeter.

In den Darstellungen der Figuren 1 und 2 sind alle Düsenlängsrichtungen (28) in einer Ebene angeordnet. Diese Ebene liegt normal zur Kolbenhubrichtung (42). Bei der dargestellten Düsenanordnung schneidet die parallel zur Düsenlängsrichtung (28) orientierte, die Eintrittsfläche (26) mit der Austrittsfläche (27) verbindende Mittellinie (29) jeder einzelnen Düse (25) die Mittellinie (31) des Zylinders (11). Beide Mittellinien (29, 31) sind Abschnitte von Geraden. Die Düse (25) umgreift hiermit zumindest eine Gerade oder eine Geradenschar, die sowohl die Eintrittsfläche (26) als auch die Austrittsfläche (27) schneidet. Es ist auch denkbar, dass die die Düse (25) durchdringende Gerade und die die Zylinder-Mittellinie (31) umfassende Gerade windschief zueinander angeordnet sind und/oder sich kreuzen.

Sowohl die Kolbenhubrichtung (42) als auch die Düsenlängsrichtung (28) sind Vektoren. Sie können jeweils unter Beibehaltung ihrer Richtung und ihres Betrags in Parallelenrichtung verschoben werden. In einer geometrischen Darstellung können die beiden Vektoren mit einem gemeinsamen Ursprung gezeichnet werden. Der Richtungsvektor der Kolbenhubrichtung (42) und der Richtungsvektor einer einzelnen Düsenlängsrichtung (28) spannen eine gemeinsame Ebene auf. In dieser Ebene beträgt der von den beiden Vektoren eingeschlossene Winkel zwischen 45 Grad und 105 Grad. Die Länge der einzelnen Düse (25) ist dann z.B. länger als die oben angegebene Länge.

Der Zylinderboden (13) ist in den Darstellungen der Figuren 1 und 2 auf der Außenseite (32) konkav ausgebildet. Beispielsweise hat er in der Schnittdarstellung der Figur 1 die Gestalt eines Halbellipsoids. Die Auflagefläche ist im zentralen Bereich ballig ausgebildet und geht am Rand stetig in den zylindrischen Bereich über.

Die Innenseite (33) des Zylinderbodens (13) hat im Ausführungsbeispiel einen umlaufenden Bodenring (34), der einen kegelstumpfförmig ausgebildeten zentralen Bereich (35) umgibt. Der Bodenring (34) hat z.B. einen ebenen Boden (36), an den die Düsen (25) angrenzen. Dieser Boden (36) ermöglicht beispielsweise den Werkzeugauslauf bei der Herstellung der Düsen (25). Gegebenenfalls kann der Boden (36) des Bodenrings (34) in einer abgerundeten Hohlkehle in die Zylinderinnenwandung (15) übergehen. Der Übergang in den zentralen Bereich (35) kann als umlaufende Kehle ausgebildet sein. Im Ausführungsbeispiel beträgt der Spitzenwinkel des gedachten Kegels 170 Grad. Die gedachte Spitze ist z.B. abgerundet ausgebildet. Beispielsweise liegt dieser Spitzenwinkel zwischen 30 Grad und 180 Grad. Bei einem Spitzenwinkel von 180 Grad kann die Innenseite (33) des Zylinderbodens (13) eben ausgeführt sein.

Der Kolben (41) ist im Ausführungsbeispiel stangenlos ausgebildet. Er kann jedoch auch mittels einer Kolbenstange geführt sein. Der dargestellte Kolben (41) hat z.B. eine plane Oberseite (43) und eine konkav ausgebildete, dem Verdrängungsraum (51) zugewandte Stirnseite (44). Im Ausführungsbeispiel ist die Stirnseite (44) des Kolbens (41) komplementär zur Innenseite (33) des Zylinderbodens (13) ausgebildet. Es ist auch denkbar, die Stirnseite (44) des Kolbens (41) als ebene Fläche auszubilden.

Im Ausführungsbeispiel hat der Kolben (41) an seiner Kolbenmantelfläche (45) eine Ringnut (46). In dieser ist ein Kolbendichtelement (47), z.B. ein Dichtungsring (47) in der Bauart eines O-Rings, gelagert. Mittels dieses Kolbendichtelements (47) ist der Kolben (41) gegen die Zylinderinnenwandung (15) angedichtet. Der Kolben (41) ist somit abgedichtet im Zylinder (11) geführt. Das Kolbendichtelement (47) kann auch manschettenförmig, in der Bauform eines Wellendichtrings, etc. ausgebildet sein. Auch ist es denkbar, mehrere Kolbendichtelemente (47) einzusetzen.

Zur Vorbereitung des Einweg- oder Einmalinjektors wird die Zylinder-Kolben-Einheit (10) beispielsweise mit einer Injektionslösung (7) befüllt. Nach dem Einsetzen des Kolbens (41) befindet sich die Injektionslösung (7) im Verdrängungsraum (51). Die Düsen (25) sind beispielsweise mitels eines Originalitätsverschlusses verschlossen. Sobald die Zylinder-Kolben-Einheit (10) in den Einweginjektor eingesetzt ist, ist dieser einsatzbereit.

Nach dem Entfernen des Originalitätsverschlusses oder der Originalitätsverschlüsse wird der Einweginjektor auf die Haut (2) des Patienten aufgesetzt. Die Haut des Patienten umfasst eine Hornhaut (3), eine unter der Hornhaut (3) liegende oberste Hautschicht (4) und weitere Hautschichten (5), die unterhalb dieser obersten Hautschicht (4) liegen. Hierbei drückt die Zylinder-Kolben-Einheit (10) die Haut (2) des Patienten ein, sodass sich die Haut (2) an die Außenseite (32) des Zylinderbodens (13) und den Düsenbereich (19) anlegt. Der Auflagering (18) liegt auf der Haut (2) auf und spannt zusammen mit dem Zylinderboden (13) die Haut (2) des Patienten. Die Düsen (25) sind verdeckt und nicht sichtbar. Der Auflagering (19) stabilisiert die Lage des Einweginjektors und der Zylinder-Kolben-Einheit (10) zur Haut (2).

Beim Auslösen des Einweginjektors schlägt beispielsweise ein Betätigungsstempel auf die Oberseite (43) des Kolbens (41) und beschleunigt diesen in der Kolbenhubrichtung (42). Die Injektionslösung (7) wird aus dem Verdrängungsraum (51) durch die Düsen (25) hindurch in die Haut (2) des Patienten gefördert. Die Injektionslösung durchdringt die Hornhaut (3) weitgehend normal zur Hautoberfläche. Hierdurch kann der Wirkstoff großflächig in die oberste Hautschicht (4) eingebracht werden. Aufgrund der sich ausbildenden Geometrie der verformten Haut (2) gelangt nur ein geringer Anteil in die darunterliegenden Schichten (5) der Haut (2). Es baut sich kein Überdruck auf, sodass keine Gefahr des Zurückfließens des Wirkstoffs (7) besteht.

Während des Kolbenhubs verkleinert der in Richtung des Zylinderbodens (13) verfahrende Kolben (41) das Volumen des Verdrängungsraums (51). Die Injektionslösung (7) wird in Richtung des Bodenrings (34) und durch die Düsen (25) hindurch verdrängt. Sobald der Kolben (41) seine untere Endlage erreicht hat, ist der Verdrängungsraum (51) nahezu rückstandsfrei entleert.

Nach dem Abschluß der Injektion kann der Einweginjektor wieder von der Haut (2) des Patienten abgenommen werden. Er kann nun entsorgt werden. Die Haut (2) des Patienten nimmt wieder ihre ursprüngliche Gestalt an.

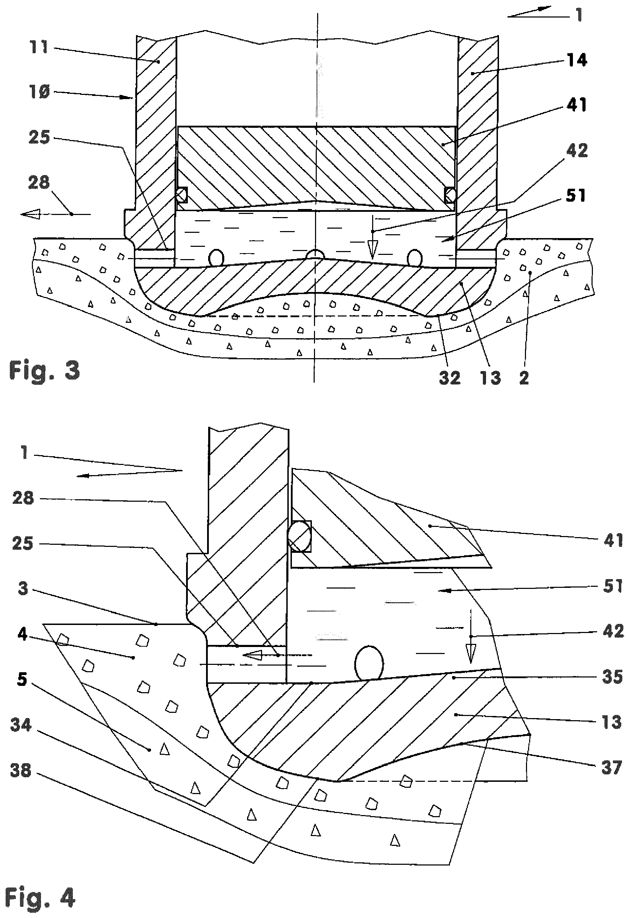

Die Figuren 3 und 4 zeigen eine weitere Ausführungsform einer Zylinder-Kolben-Einheit (10). Der Zylindermantel (14) und der Kolben (41) sind so ausgebildet, wie im Zusammenhang mit dem ersten Ausführungsbeispiel beschrieben. Der Zylinderboden (13) hat an seiner Außenseite (32) eine zentral angeordnete, konkav ausgebildete Einsenkung (37). Diese ist von einem Außenring (38) umgeben. Beispielsweise hat der Zylinderboden (13) in diesem Ausführungsbeispiel eine weitgehend gleichmäßige Wandstärke.

Der Einsatz der Zylinder-Kolben-Einheit (10) erfolgt, wie im Zusammenhang mit dem ersten Ausführungsbeispiel beschrieben. Beim Aufsetzen auf die Haut (2) des Patienten wird die Haut (2) des Patienten mittels des Außenrings (38) gespannt. Das Auslösen und das Ausbringen der Injektionsflüssigkeit (7) erfolgen, wie oben beschrieben.

Es ist auch denkbar, die verschiedenen genannten Ausführungsformen miteinander zu kombinieren.

### Bezugszeichenliste:

- 1: Umgebung
- 2: Haut
- 3: Hornhaut
- 4: oberste Hautschicht
- 5: Hautschichten unterhalb (4)

- 7: Injektionslösung, Wirkstoff, wirkstoffhaltige Lösungen

- 10: Zylinder-Kolben-Einheit
- 11: Zylinder
- 12: Zylinderinnenraum
- 13: Zylinderboden
- 14: Zylindermantel
- 15: Innenwandung, Zylinderinnenwandung
- 16: Mantelfläche
- 17: erster Abschnitt von (16), Mantelabschnitt
- 18: zweiter Abschnitt von (16), Auflagering
- 19: dritter Abschnitt von (16), Düsenbereich

- 21: Umfangsfläche
- 22: Unterseite

- 24: Hohlkehle
- 25: Düse
- 26: Eintrittsfläche
- 27: Austrittsfläche
- 28: Düsenlängsrichtung
- 29: Mittellinie von (25)

- 31: Mittellinie von (11)
- 32: Außenseite
- 33: Innenseite
- 34: Bodenring
- 35: zentraler Bereich
- 36: Boden
- 37: Einsenkung
- 38: Außenring

- 41: Kolben
- 42: Kolbenhubrichtung
- 43: Oberseite
- 44: Stirnseite
- 45: Kolbenmantelfläche
- 46: Ringnut
- 47: Dichtungsring, Kolbendichtelement

- 51: Verdrängungsraum

## Patentansprüche

1. Zylinder-Kolben-Einheit (10) für einen nadellosen Einweginjektor mit einem Zylinder (11) und mit einem in diesem abgedichtet geführten Kolben (41), wobei der Zylinder (11) und der Kolben (41) mit einem Zylinderboden (13) einen Verdrängungsraum (51) begrenzen und wobei mindestens eine Düse (25) mit einer dem Verdrängungsraum (51) zugewandten Eintrittsfläche (26) und mit einer der Umgebung (1) zugewandten Austrittsfläche (27) den Verdrängungsraum (51) mit der Umgebung (1) verbindet, wobei die Düse (25) mindestens eine Gerade umgreift, die die Eintrittsfläche (26) und die Austrittsfläche (27) schneidet und wobei die Kolbenhubrichtung (42) des Kolbens (41) in Richtung des Verdrängungsraums (51) und des Zylinderbodens (13) des Zylinders (11) orientiert ist, so dass nach Entleerung des Verdrängungsraums (51) der Kolben (41) in einer unteren Endlage am Zylinderboden (13) anliegt, **dadurch gekennzeichnet,**
- **dass** der Zylinder (11) eine Mantelfläche (16) mit einem an den Zylinderboden (13) angrenzenden Düsenbereich (19) hat, in dem der Verdrängungsraum (51) mittels der Düse (25) mit der Umgebung (1) verbunden ist,
- **dass** an den Zylinderboden (13) die Eintrittsfläche (26) angrenzt und
- **dass** die parallel zu der genannten Gerade orientierte, von der Eintrittsfläche (26) zur Austrittsfläche (27) gerichtete Düsenlängsrichtung (28) mit der Kolbenhubrichtung (42) in einer gemeinsamen Ebene einen Winkel zwischen 45 Grad und 105 Grad einschließt.

2. Zylinder-Kolben-Einheit (10) nach Anspruch 1,
**dadurch gekennzeichnet,**
**dass** die Eintrittsfläche (26) kleiner ist als die Austrittsfläche (27) oder diese beiden Flächen (26, 27) gleich groß sind.

3. Zylinder-Kolben-Einheit (10) nach Anspruch 1,
**dadurch gekennzeichnet,**
**dass** der Zylinderboden (13) eine zumindest bereichsweise konvex gewölbte Außenseite (32) aufweist.

4. Zylinder-Kolben-Einheit (10) nach Anspruch 1,
**dadurch gekennzeichnet,**
**dass** der Zylinderboden (13) einen kegelstumpfmantelförmig ausgebildeten zentralen Bereich (35) aufweist.

5. Zylinder-Kolben-Einheit (10) nach Anspruch 4,
**dadurch gekennzeichnet,**
**dass** der genannte zentrale Bereich (35) von einem Bodenring (34) mit einem ebenen Boden (36) umgeben ist.

6. Zylinder-Kolben-Einheit (10) nach Anspruch 1,
**dadurch gekennzeichnet,**
**dass** die in der Kolbenhubrichtung (42) orientierte Stirnseite (44) des Kolbens (41) komplementär zur Innenseite (33) des Zylinderbodens (13) ausgebildet ist.

7. Zylinder-Kolben-Einheit (10) nach Anspruch 1,
**dadurch gekennzeichnet,**
**dass** der Zylinder (11) an seiner Mantelfläche (16) einen Auflagering (18) aufweist, der in radialer Richtung über den Düsenbereich (19) übersteht.

8. Zylinder-Kolben-Einheit (10) nach Anspruch 7,
**dadurch gekennzeichnet,**
**dass** die Austrittsfläche (27) zwischen dem Auflagering (18) und dem Zylinderboden (13) angeordnet ist.

## Claims

1. A cylinder-piston unit (10) for a needle-free disposable injector, comprising a cylinder (11) and comprising a piston (41) guided in said cylinder in a sealed manner, wherein the cylinder (11) and the piston (41) together with a cylinder base (13) delimit a displacement space (51), and wherein at least one nozzle (25) with an entry area (26) facing the displacement space (51) and with an exit area (27) facing the surrounding environment (1) connects the displacement space (51) to the surrounding environment (1), wherein the nozzle (25) surrounds at least one straight line intersecting the entry area (26) and the exit area (27), and wherein the piston stroke direction (42) of the piston (41) is oriented in the direction of the displacement space (51) and the cylinder base (13) of the cylinder (11), such that, once the displacement space (51) has been emptied, the piston (41) rests against the cylinder base (13) in a lower end position,
**characterised in that**
- the cylinder (11) has a lateral surface (16) with a nozzle region (19) adjacent to the cylinder base (13), in which nozzle region the displacement space (51) is connected to the surrounding environment (1) by means of the nozzle (25),
- the entry area (26) is adjacent to the cylinder base (13), and
- the nozzle longitudinal direction (28) oriented parallel to the aforesaid straight line and directed from the entry area (26) to the exit area (27) encloses an angle between 45 degrees and 105 degrees with the piston stroke direction (42) in a common plane.

2. The cylinder-piston unit (10) according to claim 1,
**characterised in that**
the entry area (26) is smaller than the exit area (27), or these two areas (26, 27) are of the same size.

3. The cylinder-piston unit (10) according to claim 1,
**characterised in that**
the cylinder base (13) has an outer side (32) curved convexly at least in some regions.

4. The cylinder-piston unit (10) according to claim 1,
**characterised in that**
the cylinder base (13) has a central region (35) having the shape of a frustoconical lateral surface.

5. The cylinder-piston unit (10) according to claim 4,
**characterised in that**
said central region (35) is surrounded by a base ring (34) having a planar base (36).

6. The cylinder-piston unit (10) according to claim 1,
**characterised in that**
the end face (44) of the piston (41) oriented in the piston stroke direction (42) is formed in a manner complementary to the inner side (33) of the cylinder base (13).

7. The cylinder-piston unit (10) according to claim 1,
**characterised in that** the cylinder (11) on its lateral surface (16) has a bearing ring (18), which protrudes beyond the nozzle region (19) in the radial direction.

8. The cylinder-piston unit (10) according to claim 7,
**characterised in that**
the exit area (27) is arranged between the bearing ring (18) and the cylinder base (13).

## Revendications

1. Unité cylindre-piston (10) pour un injecteur sans aiguille à usage unique comportant un cylindre (11) et un piston (41) guidé de manière étanche dans ce cylindre,
le cylindre (11) avec le fond de cylindre (13) et le piston (41) délimitant une chambre de refoulement (51) et au moins une buse (25) avec une surface d'entrée (26) orientée vers la chambre de refoulement (51) et avec une surface de sortie (27) orientée vers l'extérieur reliant la chambre de refoulement (51) à l'extérieur,
la buse (25) entourant au moins une droite qui coupe la surface d'entrée (26) et la surface de sortie (27) et la direction de la course (42) du piston (41) étant orientée vers la chambre de refoulement (51) et le fond (13) du cylindre (11), de sorte que le piston (41), une fois que la chambre de refoulement (51) a été vidée, se trouve dans une position inférieure de fin de course en appui sur le fond du cylindre (13),
**caractérisée en ce**
- **que** le cylindre (11) possède une surface d'enveloppe (16) avec une zone de buse (19) adjacente au fond du cylindre (13) dans laquelle la chambre de refoulement (51) est reliée à l'extérieur (51) au moyen de la buse (25) .
- **que** la surface d'entrée (26) est adjacente au fond du cylindre (13) et
- **que** la direction longitudinale (28) de la buse, orientée parallèlement à la droite mentionnée ci-dessus et dirigée depuis la surface d'entrée (26) vers la surface de sortie (27), et la direction de la course du piston (42) renferment un angle entre 45 degrés et 105 degrés dans un plan commun.

2. Unité cylindre-piston (10) selon la revendication 1,
**caractérisée en ce**
**que** la surface d'entrée (26) est inférieure à la surface de sortie (27) ou que ces deux surfaces (26, 27) sont de la même grandeur.

3. Unité cylindre-piston (10) selon la revendication 1,
**caractérisée en ce**
**que** le fond du cylindre (13) présente, au moins par endroits, une face extérieure convexe.

4. Unité cylindre-piston (10) selon la revendication 1,
**caractérisée en ce**
**que** le fond du cylindre (13) présente une zone centrale (35) en forme de tronc de cône.

5. Unité cylindre-piston (10) selon la revendication 4,
**caractérisée en ce**
**que** la zone centrale mentionnée (35) est entourée d'un anneau de fond (34) avec un fond plat (36).

6. Unité cylindre-piston (10) selon la revendication 1,
**caractérisée en ce**
**que** la face frontale (44) du piston (41) orientée dans la direction de la course du piston est de forme complémentaire à la face intérieure (33) du fond du cylindre (13).

7. Unité cylindre-piston (10) selon la revendication 1,
**caractérisée en ce**
**que** le cylindre (11) possède sur sa surface d'enveloppe (16) une bague d'appui (18) qui dépasse la zone de buse (19) dans le sens radial.

8. Unité cylindre-piston (10) selon la revendication 7,
**caractérisée en ce**
**que** la surface de sortie (27) se situe entre la bague d'appui (18) et le fond du cylindre (13).
